(19) **Europäisches Patentamt / European Patent Office / Office européen des brevets**

(11) **EP 4 632 353 B1**

(12) **EUROPEAN PATENT SPECIFICATION**

(45) Date of publication and mention of the grant of the patent:
**22.04.2026 Bulletin 2026/17**

(21) Application number: **24837283.1**

(22) Date of filing: **23.02.2024**

(51) International Patent Classification (IPC):
**G01N 21/359** (2014.01)    **G16C 20/20** (2019.01)
**G16C 60/00** (2019.01)    **G01N 25/20** (2006.01)

(52) Cooperative Patent Classification (CPC):
**G01N 25/20; G01N 5/04; G16C 20/70; A24C 5/00;**
A24C 5/34; G01N 33/0098; G06F 17/00

(86) International application number:
**PCT/CN2024/078294**

(87) International publication number:
**WO 2025/175555 (28.08.2025 Gazette 2025/35)**

(54) **CIGARETTE TOBACCO BLEND COMPOSITION ANALYSIS METHOD**

VERFAHREN ZUR ANALYSE DER ZUSAMMENSETZUNG EINER ZIGARETTENTABAKMISCHUNG

PROCÉDÉ D'ANALYSE DE COMPOSITION DE MÉLANGE DE TABAC DE CIGARETTE

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(43) Date of publication of application:
**15.10.2025 Bulletin 2025/42**

(73) Proprietor: **CHINA TOBACCO YUNNAN INDUSTRIAL CO., LTD**
**Wuhua District**
**Kunming**
**Yunnan 650231 (CN)**

(72) Inventors:
• **YANG, Ji**
  **Kunming, Yunnan 650231 (CN)**
• **YANG, Jianyun**
  **Kunming, Yunnan 650231 (CN)**
• **YE, Ling**
  **Kunming, Yunnan 650231 (CN)**
• **YIN, Peipei**
  **Kunming, Yunnan 650231 (CN)**
• **ZHAO, Wei**
  **Kunming, Yunnan 650231 (CN)**
• **ZHANG, Xingyue**
  **Kunming, Yunnan 650231 (CN)**
• **JIANG, Wei**
  **Kunming, Yunnan 650231 (CN)**
• **LI, Zhenjie**
  **Kunming, Yunnan 650231 (CN)**
• **PENG, Qiyuan**
  **Kunming, Yunnan 650231 (CN)**
• **XU, Yanqun**
  **Kunming, Yunnan 650231 (CN)**
• **SU, Zhongbi**
  **Kunming, Yunnan 650231 (CN)**

(74) Representative: **Gille Hrabal**
**Partnerschaftsgesellschaft mbB**
**Patentanwälte**
**Brucknerstraße 20**
**40593 Düsseldorf (DE)**

(56) References cited:
CN-A- 105 942 567     CN-A- 107 860 868
CN-A- 110 441 187     CN-A- 114 486 616
CN-A- 114 624 143     CN-A- 116 665 811
US-A1- 2023 213 538

**Description**

TECHNICAL FIELD

The invention belongs to the technical field of tobacco, in particular to a method for analyzing the composition of cigarette leaf groups.

BACKGROUND

The quality and style characteristics of cigarettes are mainly formed by product designers through the proportions of tobacco leaves of different origins, varieties and grades. It is usually necessary to rely on formula experience and sensory evaluation, and manually select 10-20 kinds of tobacco leaves from hundreds of stock grades of tobacco raw materials to design different formulas in different proportions. As a result, the composition of the coiled-tobacco leaf groups is extremely complicated, and it is difficult to analyze the composition of the unknown coiled-tobacco leaf groups manually. It is of great significance for the analysis of unknown tobacco and the design of leaf groups to analyze the composition of coiled tobacco leaves by means of instrument testing and using objective data and scientific technology.

Thermogravimetric analysis (TG/DTA) can provide stable reaction conditions under programmed temperature conditions, and is the most ideal experimental tool for tobacco pyrolysis research. Derivative thermogravimetric methodology, also called the derivative thermogravimetric method, is derived from thermogravimetric analysis, and is a technique to record the first derivative of a TG curve with respect to temperature or time. The result of the experiment is a derivative thermogravimetric curve, that is, a DTG curve. The characteristics of DTG curves are: accurate reflection of the initial reaction temperature, maximum reaction rate temperature and reaction termination temperature of each weight loss stage; and the area of each peak on the DTG curve is proportional to the corresponding sample weight loss on the TG curve. When the TG curve is not obvious to some steps in the heating process, the DTG curve can be clearly distinguished. The main feature of thermogravimetric analysis is that it is highly quantitative and can accurately measure the mass change and the rate of change. It can be said that according to this feature, as long as the mass of a substance changes when it is heated, it can be studied by thermogravimetric analysis.

Known methods of tobacco analysis by means of thermogravimetry are described e.g. in CN107860868, CN110441187, CN116665811, CN114486616, US3033213538, CN114624143, and CN105942567.

At present, the composition analysis of coiled tobacco leaves mostly adopts the combination of tobacco chemical composition analysis, flue gas chemical composition analysis, sensory evaluation and other means. The work intensity is high, the subjectivity is strong, and the conclusions drawn are vague and not referential.

In order to solve the above problems, the invention is proposed.

SUMMARY

Since DTG curves can effectively represent the quality information of cut tobacco/tobacco leaves, the consistency between the analytical composition of the formula and the real leaf formula can be simulated and evaluated by measuring the difference in DTG curves. In order to improve the generality of cigarette tobacco leaf group analysis and the workload of analysts, the invention uses a DTG differential correlation model and a combination optimization algorithm of formula analysis to analyze and characterize the quality information of cigarette cut tobacco by using a thermal analysis atlas, which can automatically search the ratio of tobacco leaf groups in a formula, and which analyzes the composition of cigarette tobacco using objective data. The composition and proportion of leaf groups in a formula can be clearly obtained, which is of great significance to the analysis and design of leaf group formulas of competing cigarettes.

The invention provides a method for analyzing a coiled tobacco leaf group composition. The specific step is to analyze competing cigarettes (to be analyzed) and analyze their specific tobacco composition and formula ratio based on the ability to characterize tobacco quality.

The technical scheme of the invention is as follows:

A method for analyzing the composition and proportion of tobacco leaf groups in a cigarette sample based on thermal analysis, characterized in that it includes the following steps: (1) preparation of a cigarette sample to be analyzed and a single-grade tobacco leaf sample; (2) construction of a thermal analysis spectrum for the cigarette sample to be analyzed and the single-grade tobacco leaf sample; and (3) analysis of the thermal analysis spectrum to obtain the composition and proportion of tobacco leaves in the cigarette sample to be analyzed.

Preferably, in Step (1), there is a single cigarette sample to be analyzed, with at least 50 single-grade tobacco leaf samples selected. Each sample is placed in a constant temperature and humidity environment of $(22 \pm 1)$ °C and $(60 \pm 2)$ % relative humidity for at least 48 hours for equilibrium. Generally, no less than 50 typical single-grade tobacco samples are selected, and the information of the tobacco leaf samples should cover different grades, different origins and different parts, and the smoking taste of typical single-grade tobacco samples is quite different. The tobacco sample should not be less than 5 g, and the sample crushing mesh should not be less than 100 mesh.

Preferably, in Step (2), the sub-steps of collecting the thermal analysis spectrum are as follows: the samples are respectively heated in thermogravimetric (TG) crucibles by a procedure including: an initial temperature of 50 °C, heating rate of 10 °C/min; final temperature at 900 °C, and constant temperature at 900 °C for 5 min; the protection gas and reaction gas are nitrogen, and the flow rate is 20 mL/min. Taking temperature (°C) as the X-axis and mass change (%) as the Y-axis, the exported data is TG result data. Before sample analysis, the thermogravimetric analyzer is set and kept at 900 °C for 10 min to clear the impurities in the selected thermogravimetric alumina crucible, and the empty crucible is used as the reference. The instrument balance sensitivity of the thermogravimetric analyzer is not less than 0.1 μg, and the curve resolution is not less than 50 million resolution points.

According to the invention, in Step (3), the sub-steps of the analysis of the thermal analysis spectrum and obtain the composition and proportion of tobacco in the cigarette to be analyzed are:

Sub-step (A): calculating the first derivative of the obtained TG result data against time to obtain a differential weight loss DTG curve, a DTG matrix Y of cigarette samples to be analyzed, and a DTG matrix of single-grade tobacco leaves $X = [X_1 \ X_2 \ ... \ X_n]$, wherein n is the number of single-grade tobacco leaf samples;

Sub-step (B): coding formula proportion: coding the real number $R = [r_1 \ r_2 \ ... \ r_n]$ for the formula proportion of each single grade tobacco leaf, wherein n is the number of single-grade tobacco leaf samples;

Sub-step (C): randomly initializing a coding matrix R: initializing an r value to a real value between 0 and 1, wherein a sum of the values of each coding matrix should be 1, establishing a search space according to a range of more than 10 times the number of tobacco leaves composed of the formula, and randomly initializing the coding matrix, that is: $R_1$, $R_2$,..... Since the number of tobacco leaves composed of the formula is generally 10-20, the search space is established according to the 10-fold range, and 200 coding matrices are randomly initialized, namely $R_1$, $R_2$, ......$R_{200}$;

Sub-step (D): calculating a DTG matrix Z after of single-grade tobacco leaves combined according to formula ratio R, thereby obtaining 200 possible formula ratio candidates;

Sub-step (E): calculating a difference value $e$ between Z and Y using a DTG differential correlation model;

Sub-step (F): converting the difference value $e$ to a probability value P(e);

Sub-step (G): according to the probability value, screening a number of formula proportions to participate in a next iteration, randomly selecting two schemes for linear reorganization: $r^{(1)} = r_1 + a * (r_1 - r_2)$, and obtaining reorganized real number coding matrices $R_1^{(1)}$, $R_2^{(1)}$, ......, wherein $a$ is a scale factor generated by random numbers that obey a [-d, 1+d] uniform distribution, and d is a value that limits the scope of the reorganization;

Sub-step (H): repeating sub-steps (C) - (F) for iterative searching, and iteratively calculating $e^{(2)}$, $e^{(3)}$, $e^{(4)}$, $e^{(5)}$...... until e is less than a certain value; and

Sub-step (I): ordering the probability value P(e) from largest to smallest, taking a number of formula proportions, and obtaining the tobacco composition and proportion of the cigarette to be analyzed.

Preferably, sub-step (C) should ensure that the sum of the values of each encoding matrix should be 1, and the initialization formula is as follows: $r_i = r_i / \sum_{i=1}^{n} r_i$ .

Preferably, in sub-step (D), to calculate the cigarette DTG matrix Z after combining single-grade tobacco leaves according to the formula ratio R, the calculation formula is as follows: $Z_i = X' \times R_i$, where $R_i$ is the i-th random coding matrix, X is the tobacco DTG matrix, and $Z_i$ is the formula DTG matrix composed of formula proportion $R_i$.

Preferably, the formula for calculating the difference value $e$ in sub-step (E) is as follows: $e = \sqrt{(Z-Y)' \Sigma^{-1} (Z-Y)}$ , where Y is the DTG matrix of the cigarette sample to be analyzed, Z is the DTG matrix of tobacco composed of the formula proportion, and $\Sigma$ is a covariance matrix between Y and Z.

Preferably, in sub-step (F), the calculation formula to convert the difference value e to the probability value P(e) between 0 and 1 is: $P(e) = \frac{e_{max}-e}{e_{max}-e_{min}} / sum(\frac{e_{max}-e}{e_{max}-e_{min}})$ .

Preferably, d has a value of 0.2-0.3 in sub-step (G). To limit the scope of reorganization, the value of d is generally 0.25.

Preferably, in sub-step (H), *e* is iteratively calculated until it is < 0.0001.

The invention has the following beneficial effects:

1. The method of the present invention uses a DTG differential correlation model and a combination optimization algorithm for formula analysis, and automatically searches for the tobacco ratio in the cigarette formula. The composition analysis of any finished cigarette on the market to be analyzed can be completed within a few minutes, and a clear formula composition and proportion value can be obtained, which is objective, efficient, versatile, and with good repeatability and high sensitivity. It has a unique advantage in the analysis of finished cigarettes in the tobacco industry.

2. The method of the present invention avoids wet chemical analysis of a large amount of tobacco, such as in conventional flue gas chemical composition analysis of tobacco leaf groups, and turns to dry chemical operation, which has the advantages of simple operation, minimal sample usage, within 10 mg, is non-toxic and harmless, and causes no harm to the operator and no environmental pollution.

3. The method of the present invention not only provides a thermal analysis spectrum of the quality of the finished cigarettes to be analyzed and the single-grade tobacco leaves by thermal analysis, but also greatly reduces the workload and the number of tests, provides concrete formula design objectives, rich data support and digital technical means for the development of cigarette products, and realizes the automatic search and objective evaluation of formula design schemes. It can avoid subjective factors and different representations that occur in traditional reliance on expert experience and sensory evaluation.

## EXAMPLES

The present invention is further explained by embodiments below, but is not limited by the present embodiments. Experimental methods not specified in the embodiments are generally available commercially in accordance with conventional conditions, conditions described in the manual, or general equipment, materials, reagents, etc. used in accordance with conditions suggested by the manufacturer, unless otherwise specified. The following embodiments and the raw materials in the applicable ratios are commercially available.

Example: Analysis method of composition and proportion of coiled tobacco leaf group of a well-known domestic brand cigarette product sample (to be analyzed), the steps are as follows:

(1) A product sample of a well-known domestic brand of cigarettes (cigarette to be analyzed) and 50 single-grade tobacco samples of different origin, different parts and different grades of 5 grams (single-grade tobacco leaves) were selected. The cigarettes to be analyzed and single-grade tobacco leaves were screened with a 100 mesh screen and treated for 48 hours in a constant temperature and humidity environment of (22 ± 1) °C and (60 ± 2) % relative humidity.

(2) Before the sample thermogravimetric analysis, the thermogravimetric analyzer was set and kept at 900 °C for 10 min to clear the impurities in the furnace body, and the empty crucible was used as a reference. A (5.00 ± 0.05) mg sample was weighed and placed in a platinum thermogravimetric crucible, and the heating procedure was as follows: an initial temperature of 50 °C, a heating rate of 10 °C/min; a final temperature of 900 °C, and a constant temperature at 900 °C for 5 min. The protection gas and reaction gas were nitrogen, and the flow rate was 20 mL/min. Taking temperature (°C) as the X-axis and mass change (%) as the Y-axis, the exported data is TG result data.

(3) deriving the weight data by time to obtain the differential weight loss curve data (DTG matrix), a DTG matrix Y for cigarettes to be analyzed, and DTG matrices for single-grade tobacco leaves X = [$X_1$ $X_2$... $X_{50}$].

Table 1: Cigarette DTG matrix Y

| Temperature °C | 30 | 31 | ... | 45 | 46 | ... | 900 |
|---|---|---|---|---|---|---|---|
| Cigarette to be analyzed | -2.62E-05 | -2.62E-05 | ... | -2.81E-05 | -3.62E-05 | ... | -5.43E-05 |

Table 2: Single grade tobacco DTG matrix X

| Temperature °C | 30 | 31 | ... | 45 | 46 | ... | 900 |
|---|---|---|---|---|---|---|---|
| Tobacco leaf 1 ($X_1$) | -3.86E-05 | -3.86E-05 | ... | -4.07E-05 | -4.94E-05 | ... | -4.51E-05 |
| Tobacco leaf 2 ($X_2$) | -2.53E-05 | -2.53E-05 | ... | -2.73E-05 | -3.54E-05 | ... | -5.27E-05 |
| ... | ... | ... | ... | ... | ... | ... | ... |

(continued)

| Temperature °C | 30 | 31 | ... | 45 | 46 | ... | 900 |
|---|---|---|---|---|---|---|---|
| Tobacco leaf 50 ($X_{50}$) | -2.77E-05 | -2.77E-05 | ... | -2.96E-05 | -3.78E-05 | ... | -4.72E-05 |

(4) The formula ratio real number coding matrix R = [$r_1$ $r_2$ ... $r_{50}$] is set, wherein $r_1$, $r_2$... $r_{50}$ represents the proportion of 50 single-grade tobacco leaves used in the formulation, as shown in Table 3:

Table 3: Formula proportional real number coding matrix R

| Single grade tobacco | Formula ratio $r_i$ |
|---|---|
| Tobacco leaf 1 | $r_1$ |
| Tobacco leaf 2 | $r_2$ |
| ... | ... |
| Tobacco leaf 50 | $r_{50}$ |

(5) Initialize $r_i$ in the above table randomly to a real value between 0 and 1, and normalize $r_i$ to ensure that the sum of proportion values of each tobacco leaf is 1. The formula is as follows: $r_i = r_i / \sum_{i=1}^{n} r_i$ , where n is 50.

Table 4: Random initialization results of formula proportional real number coding matrix R

| Single grade tobacco | Formula ratio $r_i$ |
|---|---|
| Tobacco leaf 1 | 0.03 |
| Tobacco leaf 2 | 0.04 |
| ... | ... |
| Tobacco leaf 50 | 0.06 |
| Total | 1.00 |

(6) According to the above method, 200 real number coding matrices $R_1$, $R_2$, ......$R_{200}$ are initialized at the same time to establish the formulas to constitute an analytic search space, as shown in the following table:

Table 5: Formula proportional real number coding initialization

| Coding | $R_1$ | $R_2$ | $R_3$ | $R_4$ | ... | $R_{200}$ |
|---|---|---|---|---|---|---|
| $r_1$ | 0.05 | 0.14 | 0.02 | 0.00 | ... | 0.12 |
| $r_2$ | 0.02 | 0.02 | 0.01 | 0.16 | ... | 0.14 |
| $r_3$ | 0.04 | 0.03 | 0.02 | 0.22 | ... | 0.02 |
| $r_4$ | 0.03 | 0.05 | 0.08 | 0.06 | ... | 0.03 |
| ... | ... | ... | ... | ... | ... | ... |
| $r_{50}$ | 0.26 | 0.04 | 0.08 | 0.08 | ... | 0.09 |
| Total | 1.00 | 1.00 | 1.00 | 1.00 | 1.00 | 1.00 |

(7) A single grade tobacco DTG matrix X is combined according to the formula proportion real number coding matrix R to calculate the cigarette DTG matrix Z; $Z_i = X' \times R_i$;

Table 6: Cigarette fusion map matrix Z after combination

| Temperature °C | 30 | 31 | ... | 45 | 46 | ... | 900 |
|---|---|---|---|---|---|---|---|
| $Z_1$ | -3.23E-05 | -3.23E-05 | ... | -3.43E-05 | -4.24E-05 | ... | -5.99E-05 |

(continued)

| Temperature °C | 30 | 31 | ... | 45 | 46 | ... | 900 |
|---|---|---|---|---|---|---|---|
| $Z_2$ | -3.35E-05 | -3.35E-05 | ... | -3.54E-05 | -4.32E-05 | ... | -5.86E-05 |
| ... | ... | ... | ... | ... | ... | ... | ... |
| $Z_{200}$ | -6.27E-05 | -6.27E-05 | ... | -6.51E-05 | -7.48E-05 | ... | -5.47E-05 |

(8) A DTG difference correlation model, $e = \sqrt{(Z - Y)' \Sigma^{-1} (Z - Y)}$, is used to calculate the difference value e between Z and Y, so as to evaluate the conformity of the composition analysis of the tobacco leaf groups, as shown in Table 7 below:

Table 7: Analytical coincidence (difference between Z and Y) of coiled tobacco composition

| Candidate | $R_1$ | $R_2$ | $R_3$ | $R_4$ | ... | $R_{200}$ |
|---|---|---|---|---|---|---|
| Difference e | 3.6703 | 2.5197 | 6.2708 | 2.8521 | ... | 1.5228 |

(9) Convert the difference value *e* to a probability value P(e): $P(e) = \dfrac{e_{max}-e}{e_{max}-e_{min}} \Big/ sum\left(\dfrac{e_{max}-e}{e_{max}-e_{min}}\right)$ , as shown in Table 8 below:

Table 8: Convert the difference value e to a probability value

| Candidate | $R_1$ | $R_2$ | $R_3$ | $R_4$ | ... | $R_{200}$ | Total |
|---|---|---|---|---|---|---|---|
| Probability value P(e) | 2.38% | 4.36% | 4.79% | 3.79% | ... | 6.08% | 100% |

(10) The first 100 candidates for the formula ratio were selected randomly according to the probability values, and the formula ratios of the candidates were reorganized linearly in pairs, $r^{(1)} = r_1 + a * (r_1 - r_2)$, where a is a scale factor, generated by random numbers with a uniform distribution following [-d, 1+d], and d is 0.25, a limit so that the reorganization range is not too large.
The reorganized real number coding matrix $R_1^{(1)}$, $R_2^{(1)}$, ...... $R_{200}^{(1)}$ is obtained as shown in Table 9 below:

Table 9: Real number code of formula ratio after the first reorganization

| Code | $R_1^{(1)}$ | $R_2^{(1)}$ | $R_3^{(1)}$ | $R_4^{(1)}$ | ... | $R_{200}^{(1)}$ |
|---|---|---|---|---|---|---|
| $r_1$ | 0.00 | 0.14 | 0.40 | 0.07 | ... | 0.19 |
| $r_2$ | 0.20 | 0.03 | 0.01 | 0.05 | ... | 0.06 |
| $r_3$ | 0.25 | 0.25 | 0.08 | 0.02 | ... | 0.07 |
| $r_4$ | 0.03 | 0.39 | 0.27 | 0.13 | ... | 0.05 |
| ... | ... | ... | ... | ... | ... | ... |
| $r_{50}$ | 0.07 | 0.03 | 0.11 | 0.01 | ... | 0.07 |
| Total | 1.00 | 1.00 | 1.00 | 1.00 | 1.00 | 1.00 |

(11) According to the real number coding of the reorganized formula proportion, the reorganized cigarette fusion map matrix $Z^{(1)}$ was calculated, the DTG difference association model was invoked to calculate the difference value $e^{(1)}$ between Z and Y, and the iterative calculation of $e^{(2)}$, $e^{(3)}$, $e^{(4)}$, $e^{(5)}$,...... , until e = 0.000095 < 0.0001.
(12) According to the probability value P(e) from large to small, the top 5 formula proportional candidates are output, as shown in Table 10 below:

Table 10: Formula analysis results and P(e) values (the top 5 candidates with the highest probability values are selected)

| Code | $R_{75}^{(1)}$ | $R_{24}^{(1)}$ | $R_{13}^{(1)}$ | $R_{15}^{(1)}$ | $R_{180}^{(1)}$ |
|---|---|---|---|---|---|
| $r_1$ | 0.00 | 0.00 | 0.00 | 0.00 | 0.00 |
| $r_2$ | 0.00 | 0.03 | 0.00 | 0.08 | 0.05 |
| $r_3$ | 0.10 | 0.25 | 0.20 | 0.15 | 0.16 |
| $r_4$ | 0.15 | 0.39 | 0.18 | 0.10 | 0.27 |
| ... | ... | ... | ... | ... | ... |
| $r_{50}$ | 0.07 | 0.08 | 0.15 | 0.11 | 0.07 |
| **P(e)** | **70.30%** | **13.91%** | **4.46%** | **2.24%** | **1.15%** |

(13) According to the first five formula ratio candidates in the above table, tobacco leaves with a formula ratio of 0 are filtered out to obtain the complete composition and ratio of the tobacco leaf formula, as shown in Tables 11-15 below:

Table 11: Leaf formula corresponding to $R_{75}$

| Single grade tobacco | Formula ratio |
|---|---|
| Tobacco Leaf 3 | 0.10 |
| Tobacco Leaf 4 | 0.15 |
| Tobacco Leaf 7 | 0.05 |
| Tobacco Leaf 14 | 0.05 |
| Tobacco Leaf 18 | 0.10 |
| Tobacco Leaf 20 | 0.05 |
| Tobacco Leaf 25 | 0.10 |
| Tobacco Leaf 26 | 0.05 |
| Tobacco Leaf 27 | 0.05 |
| Tobacco Leaf 32 | 0.10 |
| Tobacco Leaf 35 | 0.05 |
| Tobacco Leaf 42 | 0.04 |
| Tobacco Leaf 46 | 0.04 |
| Tobacco Leaf 50 | 0.07 |

Table 12: Leaf formulations corresponding to $R_{24}$

| Single grade tobacco | Formula ratio |
|---|---|
| Tobacco Leaf 2 | 0.05 |
| Tobacco Leaf 3 | 0.12 |
| Tobacco Leaf 4 | 0.10 |
| Tobacco Leaf 14 | 0.05 |
| Tobacco Leaf 15 | 0.08 |
| Tobacco Leaf 20 | 0.05 |
| Tobacco Leaf 25 | 0.10 |
| Tobacco Leaf 26 | 0.05 |
| Tobacco Leaf 27 | 0.05 |
| Tobacco Leaf 32 | 0.12 |

(continued)

| Single grade tobacco | Formula ratio |
|---|---|
| Tobacco Leaf 35 | 0.05 |
| Tobacco Leaf 42 | 0.05 |
| Tobacco Leaf 46 | 0.05 |
| Tobacco Leaf 50 | 0.08 |

Table 13: Leaf formulations corresponding to $R_{13}$

| Single grade tobacco | Formula ratio |
|---|---|
| Tobacco Leaf 3 | 0.20 |
| Tobacco Leaf 4 | 0.18 |
| Tobacco Leaf 8 | 0.08 |
| Tobacco Leaf 13 | 0.02 |
| Tobacco Leaf 17 | 0.06 |
| Tobacco Leaf 19 | 0.10 |
| Tobacco Leaf 25 | 0.02 |
| Tobacco Leaf 28 | 0.05 |
| Tobacco Leaf 30 | 0.07 |
| Tobacco Leaf 35 | 0.06 |
| Tobacco Leaf 45 | 0.03 |
| Tobacco Leaf 46 | 0.05 |
| Tobacco Leaf 50 | 0.08 |

Table 14: Leaf formulations corresponding to $R_{15}$

| Single grade tobacco | Formula ratio |
|---|---|
| Tobacco Leaf 2 | 0.08 |
| Tobacco Leaf 3 | 0.15 |
| Tobacco Leaf 4 | 0.10 |
| Tobacco Leaf 8 | 0.05 |
| Tobacco Leaf 13 | 0.06 |
| Tobacco Leaf 17 | 0.06 |
| Tobacco Leaf 19 | 0.02 |
| Tobacco Leaf 25 | 0.02 |
| Tobacco Leaf 28 | 0.05 |
| Tobacco Leaf 30 | 0.08 |
| Tobacco Leaf 35 | 0.12 |
| Tobacco Leaf 45 | 0.05 |
| Tobacco Leaf 46 | 0.05 |
| Tobacco Leaf 50 | 0.11 |

Table 15: Leaf formulations corresponding to $R_{180}$

| Single grade tobacco | Formula ratio |
|---|---|
| Tobacco Leaf 2 | 0.05 |
| Tobacco Leaf 3 | 0.16 |
| Tobacco Leaf 4 | 0.27 |
| Tobacco Leaf 5 | 0.03 |
| Tobacco Leaf 12 | 0.01 |
| Tobacco Leaf 14 | 0.05 |
| Tobacco Leaf 15 | 0.02 |
| Tobacco Leaf 20 | 0.02 |
| Tobacco Leaf 25 | 0.05 |
| Tobacco Leaf 26 | 0.04 |
| Tobacco Leaf 27 | 0.04 |
| Tobacco Leaf 32 | 0.06 |
| Tobacco Leaf 35 | 0.03 |
| Tobacco Leaf 42 | 0.03 |
| Tobacco Leaf 44 | 0.02 |
| Tobacco Leaf 45 | 0.02 |
| Tobacco Leaf 46 | 0.03 |
| Tobacco Leaf 50 | 0.07 |

Verification experiment: According to the five formulations shown in Tables 11-15, the single-grade tobacco leaf samples involved were mixed into cigarettes, and 9 sensory evaluation experts evaluated and scored the sensory quality differences between the mixed tobacco sample and the cigarette sample to be analyzed. The average value was taken as the actual smoking evaluation value of the quality difference, and the quality difference was qualitative after rounding. The scoring gradient settings are shown in Table 16 below:

Table 16: Sensory quality difference score gradient setting

| Quality deviation | None | slight | lesser | Intermediate | big | Large |
|---|---|---|---|---|---|---|
| Score | 0 | 1 | 2 | 3 | 4 | 5 |

The smoking evaluation results are shown in Table 17 below:

Table 17: Verification results of sensory evaluation

| Candidate formulation | P(e) | Average | Quality difference |
|---|---|---|---|
| $R_{75}$ | 70.30% | 0.00 | None |
| $R_{24}$ | 13.91% | 0.66 | Slight |
| $R_{13}$ | 4.46% | 0.78 | Slight |
| $R_{15}$ | 2.24% | 1.11 | Slight |
| $R_{180}$ | 1.15% | 1.22 | Slight |

As can be seen from Table 17, the consistency between formula ratio candidate $R_{75}$ and cigarettes to be analyzed was 70.30%, and there was no difference in sensory evaluation results. The coincidence between candidate $R_{24}$, $R_{13}$, $R_{15}$, $R_{180}$ and the cigarettes to be analyzed were 13.91%, 4.46%, 2.24% and 1.15%, respectively, and the results of sensory evaluation were slightly different.

The above embodiments disclose only several embodiments of the invention, and their descriptions are more specific and detailed, but they cannot be construed as limitations on the scope of the invention. The scope of protection of the invention patent is defined in the attached claims.

**Claims**

1.  A method for analyzing a composition of tobacco leaves, **characterized in that** it comprises the following steps: (1) preparing cigarette samples to be analyzed and single-grade tobacco samples; (2) collecting a thermal analysis spectrum of the cigarette samples to be analyzed and the single-grade tobacco samples; (3) analyzing the thermal analysis spectrum to obtain the tobacco leaf composition and proportion of the cigarette samples to be analyzed; the sub-steps of analyzing the thermal analysis spectrum to obtain the tobacco composition and proportion of the cigarette samples to be analyzed are:

    Sub-step (A): calculating a first derivative of the TG result data against time to obtain a differential weight loss DTG curve, a DTG matrix Y of the cigarette samples to be analyzed, and a DTG matrix of the single-grade tobacco samples $X = [X_1 \ X_2 \ ... \ X_n]$, wherein n is the number of single-grade tobacco samples;
    Sub-step (B): coding formula proportion: coding a real number $R = [r_1 \ r_2 \ ... \ r_n]$ for a formula proportion of each single grade tobacco sample, where n is the number of single-grade tobacco samples;
    Sub-step (C), randomly initializing a coding matrix R: initializing a value of r to a real value between 0 and 1, where a sum of the values of each coding matrix should be 1; establishing a search space according to a range of more than 10 times a number of tobacco leaves composed of the formula, and randomly initializing the coding matrix, that is: $R_1, R_2, ......$;
    Sub-step (D), calculating a DTG matrix Z of single-grade tobacco leaves combined according to a formula ratio R;
    Sub-step (E): calculating a difference value $e$ between Z and Y using a DTG difference correlation model;
    Sub-step (F), converting the difference value e to a probability value P(e);
    Sub-step (G), according to the probability value, screening a number of formula proportions to participate in a next iteration, randomly selecting two schemes for linear reorganization: $r^{(1)} = r_1 + a * (r_1 - r_2)$, and obtaining reorganized real number coding matrices $R_1^{(1)}, R_2^{(1)}, ......$, wherein $a$ is a scale factor generated by random numbers that obey a $[-d, 1+d]$ uniform distribution, and d is a value that limits a scope of reorganization;
    Sub-step (H), repeating sub-steps (C) - (F) for iterative searching, and iteratively calculating $e^{(2)}, e^{(3)}, e^{(4)}, e^{(5)}......$ until $e$ is less than a certain value; and
    Sub-step (I), ordering the probability value P(e) from largest to smallest, taking a number of formula proportions, and obtaining the tobacco composition and proportion of the cigarette samples to be analyzed.

2.  The analytical method of the cigarette leaf group composition of Claim 1, **characterized in that** step (1) includes a single cigarette sample to be analyzed and at least 50 single-grade tobacco leaf samples; each sample is placed in a constant temperature and humidity environment of $(22 \pm 1)\,°C$ and $(60 \pm 2)\,\%$ relative humidity for at least 48 hours for equilibrium.

3.  The analytical method of the cigarette leaf group composition of claim 1, **characterized in that** in step (2), the sub-steps of collecting the thermal analysis spectrum are as follows: samples are respectively placed in thermogravimetric crucibles (TG) and heated according to a procedure including an initial temperature at 50 °C, a heating rate of 10 °C/min, a final temperature of 900 °C, and a constant temperature at 900 °C for 5 min; the protection gas and reaction gas were nitrogen, and a flow rate is 20 mL/min; taking temperature (°C) as an X-axis and mass change (%) as a Y-axis, deriving TG data, exported data is TG result data.

4.  The analytical method of the cigarette leaf group composition of claim1, **characterized in that** an initialization formula for sub-step (C) is as follows: $r_i = r_i / \sum\limits_{i=1}^{n} r_i$ .

5.  The analytical method of the cigarette leaf group composition of Claim 1, **characterized in that** in sub-step (D), to calculate the cigarette DTG matrix Z after combining single-grade tobacco leaves according to the formula ratio R, the calculation formula is as follows: $Z_i = X' \times R_i$, wherein $R_i$ is the i-th random coding matrix, X is the tobacco DTG matrix, and $Z_i$ is the formula DTG matrix composed of formula proportion $R_i$.

6. The analytical method of the cigarette leaf group composition of Claim 1, **characterized in that** a formula for calculating the difference value $e$ in sub-step (E) is as follows: $e = \sqrt{(Z - Y)'\, \Sigma^{-1}\, (Z - Y)}$, wherein Y is the DTG matrix of the cigarette samples to be analyzed, Z is the formula DTG matrix composed by proportional formula, and $\Sigma$ is a covariance matrix between Y and Z.

7. The analytical method of the cigarette leaf group composition of Claim 1, **characterized in that** in sub-step (F), a calculation formula to convert the difference value $e$ to the probability value P(e) between 0 and 1 is as follows:

$$\mathrm{P(e)} = \frac{\frac{e_{max} - e}{e_{max} - e_{min}}}{sum\left(\frac{e_{max} - e}{e_{max} - e_{min}}\right)} .$$

8. The analytical method of the cigarette leaf group composition of Claim 1, **characterized in that** d in sub-step (G) has a value of 0.2-0.3.

9. The analytical method of the cigarette leaf group composition of Claim 1, **characterized in that** in sub-step (H), $e$ is iteratively calculated until it is < 0.0001.

## Patentansprüche

1. Ein Verfahren zur Analyse einer Zusammensetzung von Tabakblättern, **dadurch gekennzeichnet, dass** es die folgenden Schritte umfasst: (1) Vorbereiten von zu analysierenden Zigarettenproben und von sortenreinen Tabakproben; (2) Sammeln eines Thermoanalysespektrums der zu analysierenden Zigarettenproben und der sortenreinen Tabakproben; (3) Analysieren des Thermoanalysespektrums, um die Tabakblatt-Zusammensetzung und den Anteil der zu analysierenden Zigarettenproben zu erhalten;
   die Unterschritte des Analysierens des Thermoanalysespektrums zu, Erhalten der Tabakzusammensetzung und des Anteils der zu analysierenden Zigarettenproben sind:

   Unterschritt (A): Berechnen einer ersten Ableitung der TG-Ergebnisdaten nach der Zeit, um eine DTG-Differenzgewichtsverlustkurve, eine DTG-Matrix Y der zu analysierenden Zigarettenproben und eine DTG-Matrix der sortenreinen Tabakproben $X = [X_1\ X_2 ... X_n]$ zu erhalten, wobei n die Anzahl der sortenreinen Tabakproben ist;
   Unterschritt (B): Codierung eines Formelanteils: Kodierung einer reellen Zahl $R = [r_1\ r_2 ... r_n]$ für einen Formelanteil jeder sortenreinen Tabakprobe, wobei n die Anzahl der sortenreinen Tabakproben ist;
   Unterschritt (C), zufällige Initialisierung einer Codierungsmatrix R: Initialisierung eines Wertes von r auf einen reellen Wert zwischen 0 und 1, wobei eine Summe der Werte jeder Codierungsmatrix 1 sein sollte; Einrichtung eines Suchraumes gemäß einem Bereich von mehr als dem 10-fachen einer Anzahl von Tabakblättern, die aus der Formel bestehen, und zufällige Initialisierung der Codierungsmatrix, das heißt: $R_1, R_2, ......$;
   Unterschritt (D): Berechnen einer DTG-Matrix Z von sortenreinen Tabakblättern, kombiniert gemäß einem Formelverhältnis R;
   Unterschritt (E): Berechnen eines Differenzwertes e zwischen Z und Y unter Verwendung eines DTG-Differenzkorrelationsmodells;
   Unterschritt (F): Umwandlung des Differenzwertes e in einen Wahrscheinlichkeitswert P(e);
   Unterschritt (G), gemäß dem Wahrscheinlichkeitswert, Screening einer Anzahl von Formelproportionen, die an einer nächsten Iteration teilnehmen sollen, zufällige Auswahl von zwei Schemata für die lineare Reorganisation: $r^{(1)} = r_1 + a * (r_1 - r_2)$, und Erhalten von reorganisierten reellen Zahlen-Codierungsmatrizen $R_1^{(1)}, R_2^{(1)}, ......$, wobei $a$ ein Skalierungsfaktor ist, der durch Zufallszahlen erzeugt wird, die einer [-d, 1+d] Gleichverteilung gehorchen, und d ein Wert ist, der einen Umfang der Reorganisation begrenzt;
   Unterschritt (H), Wiederholen der Unterschritte (C) - (F) für eine iterative Suche und iteratives Berechnen von $e^{(2)}$, $e^{(3)}$, $e^{(4)}$, $e^{(5)}......$, bis e kleiner als ein bestimmter Wert ist; und
   Unterschritt (I), Ordnen des Wahrscheinlichkeitswertes P(e) vom größten zum kleinsten, Nehmen einer Anzahl von Formelanteilen und Erhalten der Tabakzusammensetzung und des Anteils der zu analysierenden Zigarettenproben.

2. Das analytische Verfahren der Zigarettenblattzusammensetzung nach Anspruch 1, **dadurch gekennzeichnet, dass** Schritt (1) eine einzelne zu analysierende Zigarettenprobe und mindestens 50 sortenreine Tabakblattproben enthält; jede Probe wird für mindestens 48 Stunden in eine Umgebung mit konstanter Temperatur und Feuchtigkeit von (22 $\pm$

1) °C und (60 ± 2) % relativer Feuchtigkeit gebracht, um ein Gleichgewicht zu erreichen.

3. Das analytische Verfahren der Zigarettenblattzusammensetzung nach Anspruch 1, **dadurch gekennzeichnet, dass** in Schritt (2) die Unterschritte zum Sammeln des Thermoanalysespektrums wie folgt sind: Proben werden jeweils in thermogravimetrische Tiegel (TG) gegeben und gemäß einer Prozedur erhitzt, die eine Anfangstemperatur von 50 °C, eine Heizrate von 10 °C/min, eine Endtemperatur von 900 °C und eine konstante Temperatur von 900 °C für 5 min enthält; das Schutzgas und das Reaktionsgas waren Stickstoff, und der Durchfluss beträgt 20 mL/min; unter Verwendung der Temperatur (°C) als X-Achse und der Massenänderung (%) als Y-Achse werden TG-Daten abgeleitet, exportierte Daten sind TG-Ergebnisdaten.

4. Das analytische Verfahren der Zigarettenblattzusammensetzung nach Anspruch 1, **dadurch gekennzeichnet, dass** eine Initialisierungsformel für Unterschritt (C) wie folgt lautet: $r_i = r_i / \sum_{i=1}^{n} r_i$ .

5. Das analytische Verfahren der Zigarettenblattzusammensetzung nach Anspruch 1, **dadurch gekennzeichnet, dass** im Unterschritt (D) zur Berechnung der Zigaretten-DTG-Matrix Z nach der Kombination sortenreiner Tabakblätter gemäß dem Formelverhältnis R die Berechnungsformel wie folgt lautet: $Z_i = X' \times R_i$, wobei $R_i$ die i-te Zufallscodierungsmatrix, X die Tabak-DTG-Matrix und $Z_i$ die aus dem Formelverhältnis $R_i$ zusammengesetzte Formel-DTG-Matrix ist.

6. Das analytische Verfahren der Zigarettenblattzusammensetzung nach Anspruch 1, **dadurch gekennzeichnet, dass** eine Formel zur Berechnung des Differenzwertes *e im* Unterschritt (E) wie folgt lautet:

$$e = \sqrt{(Z - Y)' \Sigma^{-1} (Z - Y)}$$ , wobei Y die DTG-Matrix der zu analysierenden Zigarettenproben ist, Z die Formel-DTG-Matrix ist, die sich aus der Verhältnisformel zusammensetzt, und $\Sigma$ eine Kovarianzmatrix zwischen Y und Z ist.

7. Das analytische Verfahren der Zigarettenblattzusammensetzung nach Anspruch 1, **dadurch gekennzeichnet, dass** im Unterschritt (F) eine Berechnungsformel zur Umwandlung des Differenzwertes e in den Wahrscheinlichkeitswert P(e) zwischen 0 und 1 wie folgt lautet: $P(e) = \frac{e_{max} - e}{e_{max} - e_{min}} / sum(\frac{e_{max} - e}{e_{max} - e_{min}})$ .

8. Das analytische Verfahren der Zigarettenblattzusammensetzung nach Anspruch 1, **dadurch gekennzeichnet, dass** d im Unterschritt (G) einen Wert von 0,2-0,3 hat.

9. Das analytische Verfahren der Zigarettenblattzusammensetzung nach Anspruch 1, **dadurch gekennzeichnet, dass** in Unterschritt (H) e iterativ berechnet wird, bis es < 0,0001 ist.

## Revendications

1. Une méthode d'analyse de la composition de feuilles de tabac, **caractérisée en ce qu'**elle comprend les étapes suivantes : (1) préparer des échantillons de cigarettes à analyser et des échantillons de tabac de qualité unique ; (2) collecter un spectre d'analyse thermique des échantillons de cigarettes à analyser et des échantillons de tabac de qualité unique ; (3) analyser le spectre d'analyse thermique pour obtenir la composition des feuilles de tabac et la proportion des échantillons de cigarettes à analyser;
les sous-étapes de l'analyse du spectre d'analyse thermique pour obtenir la composition du tabac et la proportion des échantillons de cigarettes à analyser sont :

Sous-étape (A) : calculer une première dérivée des données de résultat TG par rapport au temps pour obtenir une courbe DTG différentielle de perte de poids, une matrice DTG Y des échantillons de cigarettes à analyser, et une matrice DTG des échantillons de tabac de qualité unique $X = [X_1\ X_2\ ...\ X_n]$, où n est le nombre d'échantillons de tabac de qualité unique ;
Sous-étape (B) : proportion de formule de codage : coder un nombre réel $R = [r_1\ r_2\ ...\ r_n]$ pour une proportion de formule de chaque échantillon de tabac de qualité unique, où n est le nombre d'échantillons de tabac de qualité unique ;

Sous-étape (C), initialisation aléatoire d'une matrice de codage R : initialiser une valeur de r en valeur réelle entre 0 et 1, où une somme des valeurs de chaque matrice de codage devrait être 1 ; établir un espace de recherche selon une plage de plus de 10 fois un nombre de feuilles de tabac composées de la formule, et initialiser aléatoirement la matrice de codage, c'est-à-dire : $R_1$, $R_2$, ...... ;

Sous-étape (D), calculer une matrice DTG Z de feuilles de tabac de qualité unique combinées selon un ratio de formule R ;

Sous-étape (E) : calculer une valeur de différence e entre Z et Y en utilisant un modèle de corrélation de différence DTG ;

Sous-étape (F), convertir la valeur de différence e en une valeur de probabilité P(e);

Sous-étape (G), selon la valeur de probabilité, passer au crible un nombre de proportions de formule pour participer à une itération suivante, sélectionner aléatoirement deux schémas pour une réorganisation linéaire : $r^{(1)} = r_1 + a * (r_1 - r_2)$, et obtenir des matrices de codage de nombres réels réorganisées $R_1^{(1)}$, $R_2^{(1)}$, ......, où a est un facteur d'échelle généré par des nombres aléatoires qui obéissent à une distribution uniforme [-d, 1 + d], et d est une valeur qui limite un champ de réorganisation ;

Sous-étape (H), répéter des sous-étapes (C) - (F) pour rechercher itérativement, et calculer itérativement $e^{(2)}$, $e^{(3)}$, $e^{(4)}$, $e^{(5)}$...... jusqu'à e est inférieur à une certaine valeur; et

Sous-étape (I), ordonner la valeur de probabilité P(e) de la plus grande à la plus petite, prendre un nombre de proportions de formule, et obtenir la composition et la proportion de tabac des échantillons de cigarettes à analyser.

2. La méthode analytique de la composition de groupe de feuilles de cigarette de la revendication 1, **caractérisée en ce que** l'étape (1) inclut un seul échantillon de cigarette à analyser et au moins 50 échantillons de feuilles de tabac de qualité unique ; chaque échantillon est placé dans un environnement à température et humidité constantes de $(22 \pm 1)$ °C et $(60 \pm 2)$ % d'humidité relative pendant au moins 48 heures pour l'équilibre.

3. La méthode analytique de la composition de groupe de feuilles de cigarette de la revendication 1, **caractérisée en ce que**, en étape (2), les sous-étapes de la collecte du spectre d'analyse thermique sont les suivantes : les échantillons sont respectivement placés dans des creusets thermogravimétriques (TG) et chauffés selon une procédure comprenant une température initiale de 50 °C, un taux de chauffage de 10 °C/min, une température finale de 900 °C, et une température constante à 900 °C pendant 5 minutes; le gaz de protection et le gaz de réaction sont de l'azote, et un débit est de 20 mL/min ; prendre la température (°C) comme axe X et la variation de masse (%) comme axe Y, dériver des données TG, des données exportées sont des données de résultat TG.

4. La méthode analytique de la composition de groupe de feuilles de cigarette de la revendication 1, **caractérisée en ce qu'**une formule d'initialisation pour la sous-étape (C) est la suivante : $\eta = \eta / \sum_{i=1}^{n} \eta$ .

5. La méthode analytique de la composition de groupe de feuilles de cigarette de la revendication 1, **caractérisée en ce que**, en sous-étape (D), pour calculer la matrice DTG de cigarette Z, après combinaison des feuilles de tabac de qualité unique selon le ratio de formule R, la formule de calcul est la suivante : $Z_i = X' \times R_i$, où $R_i$ est la i-ème matrice de codage aléatoire, X est la matrice DTG de tabac, et $Z_i$ est la matrice DTG de formule composée de la proportion de formule $R_i$.

6. La méthode analytique de la composition de groupe de feuilles de cigarette de la revendication 1, **caractérisée en ce qu'**une formule pour calculer la valeur de différence e en sous-étape (E) est la suivante : $e = \sqrt{(Z - Y)' \Sigma^{-1} (Z - Y)}$ , où Y est la matrice DTG des échantillons de cigarette à analyser, Z est la matrice DTG de formule composée par une formule proportionnelle, et $\Sigma$ est une matrice de covariance entre Y et Z.

7. La méthode analytique de la composition de groupe de feuilles de cigarette de la revendication 1, **caractérisée en ce que**, en sous-étape (F), une formule de calcul pour convertir la valeur de différence e en la valeur de probabilité P(e) entre 0 et 1 est la suivante : $P(e) = \dfrac{\frac{1}{e - e_{min}}}{sum(\frac{1}{e - e_{min}})}$ .

8. La méthode analytique de la composition de groupe de feuilles de cigarette de la revendication 1, **caractérisée en ce que**, en sous-étape (G), d a une valeur de 0,2 - 0,3.

9. La méthode analytique de la composition de groupe de feuilles de cigarette de la revendication 1, **caractérisée en ce que**, en sous-étape (H), e est calculé itérativement jusqu'à atteindre < 0,0001.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 107860868
- CN 110441187
- CN 116665811
- CN 114486616

- US 3033213538 A
- CN 114624143
- CN 105942567